# EUROPÄISCHE PATENTANMELDUNG

(11) **EP 1 123 694 A2**
(43) Veröffentlichungstag der Anmeldung: **16.08.2001**
(21) Anmeldenummer: 01101912.2
(22) Anmeldetag: 27.01.2001
(51) Int. Cl.: A61K 7/16

(54) **Mund- und Zahnpflegemittel**

(30) Priorität: 10.02.2000 DE 10005831
(71) Anmelder: Clariant GmbH, 65929 Frankfurt am Main (DE)
(72) Erfinder: Henning, Torsten, Dr., 65779 Kelkheim (DE); Turowski-Wanke, Angelika, Dr., 65779 Kelkheim (DE); Johannpeter, Wiebke, 61449 Steinbach (DE)

(57) **Zusammenfassung**

Mund- und Zahnpflegemittel, enthaltend Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

## Beschreibung

Die Erfindung betrifft oberflächenaktive Mittel, insbesondere Mund- und Zahnpflegemittel mit einem ausgewählten Gehalt an Sorbitolestern. Die Produkte weisen eine ausgezeichnete Geschmacks- und Geruchsqualität auf und sind dermatologisch gut verträglich.

In DE 199 06 368.0 werden Mischungen aus offenkettigen Sorbitmonoestern, -diestern und Partialglyceriden mit geringen Mengen an cyclischen Sorbitesteranteilen beschrieben, die eine sehr effektive Wirkung auf die Erniedrigung der Grenzflächenspannung von sowohl polaren, als auch unpolaren Flüssigkeiten haben, eine hohe Stabilität der Emulsionen auch bei Temperaturbelastung bewirken, eine geringe Empfindlichkeit gegenüber Elektrolyten und Säuren zeigen und sehr hautfreundlich sind.

Überraschenderweise wurde nun gefunden, dass die dort beschriebenen Sorbitester, die durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte hergestellt werden, hervorragende Solubilisiermittel von Aromastoffen und Parfümölen in Zahn- und Mundpflegeprodukten sind und darüber hinaus als Konsistenzregler wirken. Ein wesentlicher Vorteil der erfindungsgemäß in Mund- und Zahnpflegemittel eingesetzten Sorbitester ist deren ausgezeichnete Geschmacks- und Geruchsneutralität.

Gegenstand der Erfindung sind Mund- und Zahnpflegemittel, enthaltend offenkettige Sorbitester, hergestellt durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

Die erfindungsgemäß in Mund- und Zahnpflegemittel eingesetzten Sorbitester werden hergestellt durch Reaktion von Sorbit mit Fettsäuremethylestern oder Fettsäuretriglyceriden entsprechend dem in DE 197 27 950 beschriebenen Verfahren, wobei als Katalysatoren hier allerdings übliche alkalische Katalysatoren, insbesondere Natriummethylat eingesetzt werden. Der Fettsäurerest in den Fettsäuremethylestern und Fettsäuretriglyceriden enthält im allgemeinen 8 bis 22 C-Atome und kann geradkettig oder verzweigt, gesättigt oder ungesättigt sein. Beispiele hierfür sind Palmitinsäure, Stearinsäure, Laurinsäure, Linolsäure, Linolensäure, Isostearinsäure oder Ölsäure. Als Fettsäuretriglyceride kommen alle nativen tierischen oder pflanzlichen Öle, Fette und Wachse in Frage, beispielsweise Olivenöl, Rüböl, Palmkernöl, Sonnenblumenöl, Kokosöl, Leinöl, Ricinusöl, Sojabohnenöl, gegebenenfalls auch in raffinierter oder hydrierter Form. Da diese natürlichen Fette, Öle und Wachse normalerweise Mischungen von Fettsäuren mit unterschiedlicher Kettenlänge darstellen, gilt dies auch für die Fettsäurereste in den erfindungsgemäß eingesetzten Sorbitolestern.

Die Umsetzung von Sorbit mit den Fettsäuretriglyceriden oder Methylestern geschieht im Eintopfverfahren ohne Lösemittel bei Temperaturen von ungefähr 120 - 140°C in Gegenwart eines alkalischen Katalysators. Die Reaktionszeit beträgt im allgemeinen 12 bis 13 Stunden. Bei der Verwendung von Fettsäuremethylester wird während dieser Reaktion das entstehende Methanol abdestilliert. Da Sorbit üblicherweise als wässrige Lösung im Handel ist, muss zunächst das Wasser entfernt werden. Dies geschieht durch Destillation bei maximal 120°C unter vermindertem Druck. Das Molverhältnis von Sorbit zu Fettsäuremethylester beträgt im allgemeinen 1:1 bis 1:2. Bei Verwendung von Fettsäuretriglyceriden beträgt das Molverhältnis im allgemeinen 1 mol Sorbit auf 3,5 bis 4,5 mol Fettsäuretriglycerid.

Die Umsetzungsprodukte können auch alkoxyliert sein, vorzugsweise ethoxyliert, der Gehalt an Ethoxylatgruppen kann 1 bis 90 -CH₂CH₂O-Gruppen auf ein Molekül Sorbit betragen. Die Einführung der Alkoxylatgruppen kann erfolgen durch eine Alkoxylierung des Sorbits nach an sich bekannten Verfahren vor der Umesterungsreaktion. Bei der Umesterung mit Fettsäuremethylestern arbeitet man jedoch vorzugsweise so, dass man zunächst die Umesterung durchführt und anschließend nach an sich bekannten Verfahren die Alkoxylatgruppen einführt.

Das Reaktionsprodukt dieser Umesterungsreaktion besteht neben Restmengen an nicht umgesetztem Sorbit im wesentlichen aus den Sorbitmonofettsäureestern und den Sorbitdifettsäureestern. Die entsprechenden Triester entstehen nur in untergeordneten Mengen. Bei der Verwendung von Fettsäuretriglyceriden als Ausgangsprodukt enthält das Reaktionsprodukt auch noch Mono- und Di-Fettsäureglycerid sowie nicht umgesetztes Triglycerid in Abhängigkeit von dem jeweils gewählten Molverhältnis der Ausgangsverbindungen.

Die so erhaltenen Mischung der verschiedenen Reaktionsprodukte eignet sich sehr gut als Solubilisiermittel, insbesondere von Aromakomponenten und als Konsistenzgeber in Mund- und Zahnpflegemittel, insbesondere Zahnpasten und Zahnputzgele, Mundwasser und Gebissreinigungsmitteln.
Die erfindungsgemäß eingesetzten Mittel werden den Mund- und Zahnpflegemitteln in den Gewichtsmengen 0,1 bis 10 %, bevorzugt 0,5 bis 5 %, besonders bevorzugt 1 bis 3 % zugesetzt.

Die erfindungsgemäßen Mund- und Zahnpflegemittel können neben den genannten Sorbitestern Geschmacks- und Aromastoffe, nichtionische und/oder anionische Tenside, Schleif- und Poliermittel, Süßungsmittel, Feuchthaltemittel, Konsistenzregler, desodorierende Wirkstoffe, Wirkstoffe gegen Mund- und Zahnerkrankungen, und wasserlösliche Fluorverbindungen enthalten.

Für Mund- und Zahnpflegemittel geeignete Geschmacks- und Aromastoffe sind beispielsweise Pfefferminzöl, Krauseminzöl, Anisöl, Sternanisöl, Kümmelöl, Eukalyptusöl, Fenchel, Zimtöl, Nelkenöl, Geraniumöl, Salbeiöl, Pimentöl, Thymianöl, Majoranöl, Basilikumöl, Citrusöl, Gaultheriaöl oder eine oder mehrere daraus isolierte oder synthetisch erzeugte Komponenten dieser Öle, wie z.B. Menthol, Carvon, Anethol, Cineol, Eugenol, Zimtaldehyd, Caryophyllen, Geraniol, Citronellol, Linalool, Salven, Thymol, Terpinen, Terpineol, Methylchavicol und Methylsalicylat. Weitere geeignete Aromen sind z.B. Methylacetat, Vanillin, lonone, Linalylacetat, Rhodinol und Piperiton.
Die erfindungsgemäßen Mittel enthalten Aromakomponenten in den Gewichtsmengen 0,1 bis 10 %, bevorzugt 0,5 bis 5 %, besonders bevorzugt 0,8 bis 2 %, bezogen auf das fertige Mittel.

Als nichtionischen Tensiden kommen in Betracht Alkyl- und/oder Alkenyloligoglykoside, Fettsäure-N-Alkylpolyhydroxyalkylamide, insbesondere Fettsäure-N-Methylglycosid, Sucroseester, Fettalkoholpolyglycolether, Alkylphenolpolyglycolether, Fettsäurepolyglykolester, Fettsäureamidpolyglycolether, Fettaminpolyglycolether, alkoxylierte Triglyceride, Fettsäureglucamide, Polyolfettsäureester, Zuckerester, Sorbitanester und Polysorbate und/oder alkoxylierten Fettalkohole.
Bevorzugte anionische Tenside sind Alkylsulfate, Alkenylsulfate, Alkylbenzolsulfonate, Alkansulfonate und sek. Alkansulfonate, Olefinsulfonate, Alkylethersulfonate, Glycerinethersulfonate, α-Methylestersulfonate, Sulfofettsäuren, Fettalkoholethersulfate, Glycerinethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarkosinate, Fettsäuretauride, Acyllactate, Alkyloligoglucosidsulfate und Alkyl(ether)phosphate, C₈-C₂₀-Fettsäure-alpha-methylestersulfonate und Alkylethersulfate.
Die erfindungsgemäßen Mittel können Tenside in den Gewichtsmengen von 0,1 bis 12 %, vorzugsweise 0,5 bis 8 %, besonders bevorzugt 1 bis 3 % bezogen auf das fertige Mittel enthalten.

Als Schleif- und Poliermittel können die erfindungsgemäßen Mittel Kreide, Dicalciumphosphat, unlösliches Natriummetaphosphat, Aluminiumsilikat, Schichtsilikate, Hydrocalcite, Calciumpyrophosphat, feinteilige Kunstharze, Kieselsäuren, Aluminiumoxid, Aluminiumoxidtrihydrat, Talkum, Zeolithe, Magnesiumaluminiumsilkat, Calciumsulfat, Magnesiumcarbonat und/oder Magnesiumoxid enthalten.
In den erfindungsgemäßen Mitteln können Schleif- und Poliermittel in den Gewichtsmengen von 2 bis 40 %, bevorzugt 5 bis 30 %, besonders bevorzugt 8 bis 15 % enthalten sein.
Als Süßungsmittel eignen sich natürliche Zucker wie Sucrose, Maltose, Lactose, Dextrose und Fructose oder synthetische Süßstoffe wie z.B. Saccharin-Natriumsalz, Natriumcyclamat, D-Tryptophan oder Aspartam.
Süßungsmittel werden in den erfindungsgemäßen Mitteln in Gewichtsmengen von 0,005 bis 2 % eingesetzt.
Als Feuchthaltemittel kommen Sorbit, Glycerin und Polyglycole, beispielsweise Polyglycole 200 USP bis 4000 in Betracht. Feuchthaltemittel werden in den erfindungsgemäßen Mitteln in den Gewichtsmengen 1 bis 95 %, bevorzugt 30 bis 65 % eingesetzt.

Die erfindungsgemäßen Mittel zeichnen sich durch eine ausgezeichnete Geschmacks- und Geruchsqualität auf und sind dermatologisch gut verträglich.
Die folgenden Beispiele sollen den Gegenstand der Erfindung näher erläutern, ohne ihn darauf einzuschränken.

### Beispiele

| Zahnputzgel | |
|---|---|
| ® Tylose C 300 P2 | 0,40 |
| Sorbit (70 % in Wasser) | 70,00 |
| ® Emulsogen SRO | 2,00 |
| PEG-32 | 3,00 |
| Wasser | 4,64 |
| Natriumsaccharin | 0,07 |
| Natriumfluorid | 0,24 |
| Aromastoff | 1,10 |
| PHB-Ethylester | 0,15 |
| Schichtsilikat/Abrasivstoff | 11,00 |
| ® Carbopol 980 | 6,00 |
| Natriumlaurylsulfat | 1,40 |

| 2 in 1 Zahnpasta | | |
|---|---|---|
| PEG-8 | 3,0 | A |
| Xanthan Gum | 0,5 | |
| Sorbit (70 % in Wasser) | 15,00 | |
| Glycerin | 55,00 | |
| Wasser | 10,86 | |
| Schichtsilikat/Abrasivstoff | 9,00 | |
| Süßstoff | 0,18 | |
| Konservierungsmittel | 0,2 | |
| Farbstoff | 1,25 | |
| Natriumlaurylsulfat | 1,2 | |
| | | |
| Emulsogen SRO | 3,00 | B |
| Aromastoff | 0,80 | |
| Menthol | 0,01 | |

Aroma in Emulsogen SRO einarbeiten und dann in die Mischung A unter Vakuum einrühren und homogenisieren.

| Gel Zahnpasta | | |
|---|---|---|
| Sorbit (70 % in Wasser) | 43,00 | A |
| | | |
| Emulsogen SRO | 2,00 | |
| PEG-32 | 5,00 | |
| Natriummonofluorphosphat | 1,12 | |
| Süßstoff | 0,20 | |
| Trinatriumphosphat | 0,10 | |
| Wasser | 22,15 | |
| A lösen und zum Mischer geben | | |
| Abrasivstoff: Silikat | 10,00 | B |
| Kieselgel | 8,00 | |
| Tylose C 300 P2 | 0,90 | |
| Titandioxid | 0,50 | |

B vormischen und zum Mixer geben 15 Minuten bei 25 bis 30°C unter Vakuum mischen. Vakuum aufheben

| | | |
|---|---|---|
| Natriumlaurylsulfat | 1,50 | C |
| Wasser | 5,53 | |

C lösen und zum Mischer geben

Alle Prozentangaben in den Anwendungsbeispielen 1 bis 6 sind Gewichtsprozente.

### Verzeichnis der eingesetzten Produkte

- Tylose C 300 P2 :: Carboxymethylcellulose
- Carbopol 980:: Polyacrylsäure

Das Produkt Emulsogen SRO (Sorbitolester des Rapsöls) wurde wie folgt hergestellt:
1 Mol Sorbit in Form eines 70 %igen Sorbitsirups wurden vorgelegt und das Wasser im Wasserstrahlvakuum bei 120°C abdestilliert. Nach Zugabe von 1 Gew.-% (bezogen auf die gesamte Einwaage) Kaliumcarbonat (30 %ig in Wasser bei 80°C) wurde bei vollem Wasserstrahlvakuum das Wasser abdestilliert. Anschließend wurden 4 Mol raffiniertes Rapsöl zugegeben und 8 Stunden bei 140°C gerührt.

## Patentansprüche

1. Mund- und Zahnpflegemittel, enthaltend Sorbitolester, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.

2. Mund- und Zahnpflegemittel nach Anspruch 1, enthaltend Sorbitolester, erhalten durch Umsetzung von Sorbit mit Fettsäuretriglyceriden.

3. Mund- und Zahnpflegemittel nach den Ansprüchen 1 und 2, dadurch gekennzeichnet, dass sie Sorbitolester in den Gewichtsmengen 0,1 % bis 10 %, bevorzugt 0,5 bis 5 %, besonders bevorzugt 1 bis 3 % enthalten.

4. Verwendung von Sorbitolestern, erhalten durch Umesterung von gegebenenfalls oxalkyliertem Sorbit mit Fettsäuremethylestern oder Fettsäuretriglyceriden und gegebenenfalls Oxalkylierung der durch Umesterung mit Fettsäuremethylestern erhaltenen Reaktionsprodukte.
